# EUROPEAN PATENT SPECIFICATION

(11) **EP 0 595 506 B1**
(45) Date of publication and mention of the grant of the patent: **30.08.2000**
(21) Application number: 93308210.9
(22) Date of filing: 14.10.1993
(51) Int. Cl.: G01N 33/483, G01N 15/10, G01N 21/00

(54) **Automated detection of cancerous or precancerous tissue by measuring malignancy associated changes**
Automatische Detektion von kanzerogenem oder vorkanzerogenem Gewebe durch Messung maligner Veränderungen
Détection automatique de tissus cancéreux ou précancéreux en mesurant des changements malignes

(30) Priority: 14.10.1992 US 961596
(43) Date of publication of application: 04.05.1994
(73) Proprietor: Oncometrics Imaging Corp., Vancouver, British Columbia V5Z 4C2 (CA)
(72) Inventor: MacAulay, Calum, Vancouver, British Columbia V5W 3E1 (CA); Palcic, Branko, Vancouver, British Columbia V6N 1B8 (CA); Garner, David, Vancouver, British Columbia V6P 2W5 (CA); Harrison, Alan, Vancouver V6S 1T8 (CA); Jaggi, Bruno, Vancouver V6K 1M8 (CA)
(74) Representative: Carmichael, David Andrew Halliday

(56) References cited:
- WO-A-85/03131
- WO-A-91/11701
- DE-A- 3 013 546
- FR-A- 2 410 270
- GB-A- 2 125 542
- US-A- 3 984 683

## Description

### FIELD OF THE INVENTION

The invention relates to apparatus and a method for automatically detecting the presence of a cancerous or precancerous lesion in a tissue by measuring the characteristics of the cell nuclei of ostensibly normal tissue.

### BACKGROUND OF THE INVENTION

For at least five decades, attempts have been made to develop a device which can automatically detect cancerous or precancerous cells deposited on a microscopy slide. For example, research and development has been undertaken by numerous groups who have been attempting to develop a fully automated prescreening device for cervical samples. In all cases, these devices attempt to perform tasks similar to those performed by human experts, i.e. scanning the entire slide at coarse and fine spatial resolutions and seeking and characterising cancerous or precancerous cells which are exfoliated from a cancerous or precancerous lesion.

Prior art devices use nuclear and cytoplasmic features such as nuclear size, nuclear shape, nuclear to cytoplasmic ratio, DNA amount in the nucleus and DNA distribution in the nucleus to identify cancerous or precancerous cells, as referred to in Bacus JW and LJ Grace - Optical microscope system for standardized cell measurements and analyses - Applies Optics 1987; 26:3280-3293 and Auffermann W and A Bocking - Early detection of precancerous lesions in dysplasias of the lung by paid DNA image cytometry - Anal Quant Cytol Histol 1985 Sep; 7(3):218-226. Many different approaches have been tries. To date no effective system is commercially available although several groups have claimed to have achieved a semi-automated version with good to excellent results. Such systems are typically composed of an automated image cytometry device having a microscope equipped with a light transducer, often a video camera, a motorized x, y, z stage under computer control, a computer with imaging board(s), and peripheral devices such as video monitors, printers and input devices. Algorithms have also been previously developed for the automated recognition of cells and nuclei, for segmentation of areas of interest, automated focus and other functions. There are a variety of approaches by which such systems operate to recognize cancerous or precancerous cells including multivariate analysis (e.g. discriminant function analysis), decision trees, and neural networks.

All systems available or under development today rely on the detection and exact characterization of at least one fully cancerous or precancerous cell. This requires very high precision, sensitivity and specificity. The known systems are plagued with the problem of inadequacy of the artifact rejection algorithms developed to date and the only way around the problem is to use human experts to differentiate between true cancerous or precancerous cells and artifacts resulting in only a semi-automated approach.

These effects have therefore not resulted in reliable detection systems. It has been well established, for example, that the false negative rate for cervical samples is between 7 - 10 % even at the best cytology screening laboratories. Additional false negative results arise even where neither the screening cytotechnicians and cytopathologists nor their equipment are at fault. This arises when the sample to be examined is taken elsewhere than from the lesion itself, for example from an area slightly removed from the spot where cancerous or precancerous growth was present. The false negative rate due to such sampling error is even greater and has been estimated to be between 10 - 20 %.

It has been reported for several tissues (e.g. cervix, colon) that very careful measurements of nuclear features of ostensibly normal cells growing in the vicinity of the cancerous growth, show slight differences from the nuclear features of truly normal cells, i.e. from the cells of a normal, healthy individual without cancerous growth in that tissue, as discussed in Bibbo N, Montag AG, Lerma-Puertes E, Dytch HE, Leelakusolvong S, Bartels PH - Karyometric marker features in tissue adjacent to invasive cervical carcinomas - Analyt Quant Cytol Histol 1989; 11:281-285 and Bibbo M, Michelassi F, Bartels PH, Dytch H, Bania C, Lerma E, Montag AG - Karyometric market features in normal-appearing glands adjacent to human colonic adenocarcinoma - Cancer Res 1990; 50:147-151. The changed nuclear features of ostensibly normal cells growing in the vicinity of the cancerous or precancerous lesion compared to those of normal cells from the same type of tissue of a normal, healthy individual are referred to in the literature as Malignancy Associated Changes (MACs) as referred to in Haroske G, Bergander S, Konig R, Meyer W - Application of malignancy-associated changes of the cervical epithelium in a hierarchic classification concept - Anal Cell Pathol 1990 Apr; 2(3):189-198. MACs particularly manifest in nuclear features, particularly those describing the distribution of the generic material in the nuclei of these cells. Although no individual feature is sufficiently discriminating to be able to distinguish between healthy individuals and those harbouring the cancerous growth, a combination of many features in a multivariate analysis has been found to provide adequate separation between such individuals. However, no fully automated system has been developed which can perform such detection. The data (images) of cells must be obtained at the highest spatial and photometric resolution, at the precise focus and exact segmentation of the areas of interest, i.e. the nucleus. It has often been said that malignancy associated changes could never be used for automated prescreening due to these requirements. It has been necessary for a highly trained expert to identify individual cells or nuclei to be examined, due to poor artifact rejection and the limited segmentation capabilities of prior art systems. In addition, a very large number of such cell images must be analysed to achieve reasonable results, typically over 200 cell images per sample.

Such a procedure is not only time consuming and tedious, it is also impractical for prescreening as it takes typically up to several hours to accumulate, capture and analyse sufficient numbers of cell images.

This invention described an apparatus and a method for measuring MACs in a fully automated way, using only images of the nuclei of ostensibly normal cells such that several hundred cell images are used in the analysis lasting only a few minutes.

### SUMMARY OF THE INVENTION

Accordingly in one of its aspects, the invention includes a microscope having a high sampling density light transducer optically connected to the primary images plane, and an imaging board associates with the transducer to capture and process digitized images from the light transducer; characterised in that the computer controls the microscope and imaging board to detect malignancy associates changes by :
(a) controlling the microscope to acquire a precise and reproducible focus of a nucleus by maximizing contrast of the nuclear material as a function of focal position;
(b) obtaining an image of a cell nucleus;
(c) correcting the image of the cell nucleus for imperfections caused by the microscope;
(d) comparing the pixels in the corrected image with a threshold to obtain a first approximation of the real edge of the nucleus;
(e) determining a real edge of the nucleus by step by step removal of pixels with the least gradient so as not to break a continuous contour around the nucleus;
(f) measuring nuclear features of the cell nucleus in the corrected image; and
(g) analysing the measured features using a multivariate analysis to detect the presence of malignancy associated changes.

In another of its aspects, the invention comprises a method of detecting malignancy associates changes in cells comprising the steps of :
(a) providing a microscope having a high sampling density light transducer optically connected to the primary image plane of the microscope and an imaging board for processing images from the light transducer;
(b) acquiring precise and reproducible focus of a nucleus in the image from the microscope by maximizing contrast of the nuclear material as a function of focal position;
(c) thresholding the image such that the threshold mask includes at least all of the edges of the nucleus to obtain a first approximation of the real edge of the nucleus;
(d) determining the real edge of the nucleus by step by step removal of pixels with the least gradient so as not to break a continuous contour around the nucleus;
(e) analysing features of the image including DNA distribution of the cell nuclei; and
(f) performing multivariate analysis of the image
(f) performing multivariate analysis of the image of the nucleus comprising cluster analysis leading to a decision tree composed of thresholds and discriminant functions.

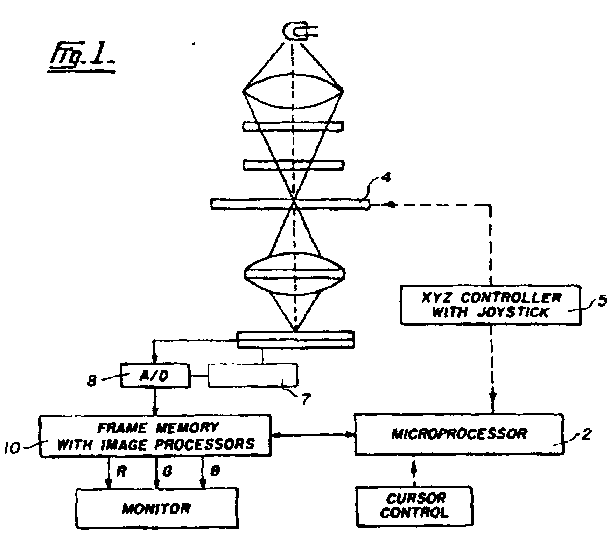

Aspects of the present invention are illustrated, merely by way of example, in the accompanying Figure 1 showing a schematic diagram of the apparatus of the invention.

### DETAILED DESCRIPTION OF THE INVENTION

The invention may be more fully appreciated by reference to the preferred embodiment thereof which relates to detection of malignancy associated changes in cervical samples.

The samples are first treated by stoichiometric staining of the nuclear (DNA) material. we achieved this by developing a modified Feulgen procedure using Thionin although other stains such as stoichiometric absorbance stains (e.g. gallocyanin, azure-A, etc.) or stoichiomeric fluorescence stains (e.g. DAPI, propidium iodide, etc.) can also be used.

Typically, a 40x objective would be used to view cells of cervical samples. However, we have found that a 40x objective results in difficulties in focusing and therefore in segmenting images of the nuclei due to the distortion effect in the lenses. In addition, the 40x objective is bulky and risks hitting the slide cover slip as well as having a shallow depth of focus. Accordingly, the preferred embodiment of the invention uses a 20x/.75 objective together with a high sampling density sensor mounted on a microscope.

A computer 2 is provided to control a motorized microscope stage 4 through controller 5 and to perform all software and output functions.

A light transducer 6, controller 7, and digitizing circuit 8 are used. Light transducer 6 consists of a scientific Charge Coupled Device with a 100% fill factor, over 500 gray level photometric resolution with a pixel size of 0.3 µm or less. The transducer is mounted in the primary image plans or in another camera port of the microscope providing negligible image distortion, and an imaging board 10 is provided for capturing and processing the images of cell nuclei.

In order to detect MACs, the device must be capable of capturing images in precise and reproducible focus. This focus is achieved by maximizing contrast of the nuclear material as a function of focal position. Given the pixel size and spatial resolution, the image of a typical nucleus comprises several hundred individual pixels of 3 - 7 µm in diameter. This is achieved with the 20x objective with the transducer placed in the primary image plane of the objective.

In addition, all images must be segmented exactly such that all pixels covering the nucleus belong to the mask. The segmentation is an important step. A simple thresholding on an image is obtained from a calibrated image (corrected for lens, illumination, dark current of camera, and other imperfections) and is corrected by the absorbance of material around the nucleus (i.e. cytoplasm) which is assumed to be present in about equal amounts over the nucleus. The thresholded mask edge represents the first approximation of the real edge of the nucleus. The latter is obtained by an edge relocation algorithm which operates by dilating the approximate edge and then step by step removing the pixels with the least gradient such as not to break a continuous contour around the nucleus.

Algorithms selecting only images of the nuclei of normal cells are used to ensure that only a few percent (typically 1 - 2%) of artifacts are present in the population of the ostensibly normal cells. This is achieved by discriminant function analysis and a decision tree process, but could possibly also be achieved by other statistical or neural network procedures, as will be appreciated by those skilled in the art of automated image classification.

Various discriminant features of the segmented digital images are then analyzed by the computer. For the MAC approach to work, depending on the cell type many nuclear features must be employed, but particularly the DNA distribution of the cell nuclei. We typically measure over 100 nuclear features of which about 30 are then used in the multivariate analysis. For best results, the nuclear mask is adjusted depending on features. For example, for features describing the frequency content of the optical density in the nucleus the pixels covering the edge of the nucleus must be removed as otherwise these features lose (or are diminished in) their discriminant power. Conversely, other features require all edge pixels, even those catching only a fraction of the material in the edge, to be included (e.g. integrated optical density). Therefore, the nuclear mask is adjusted individually for all critical features by either appropriate erosion or dilation algorithm of the best (highest gradient) edge.

Multivariate analysis may then be performed by the computer on the features of the segmented images. In the preferred embodiment, the multivariate analysis comprises cluster analysis leading to a decision tree made up of thresholds and discriminant functions. Alternatively, it can consist of a neural network. We have found that, using the above approach, reliable characterization of MACs has been achieved.

When all the above is achieved, MAC values of ostensibly normal cells are obtained which are significantly different from tissues of those individuals who are harbouring cancerous (such as carcinoma in situ, microinvasive or invasive cancer) or precancerous lesion (such as moderate and severe dysplasia). Several tissues have been examined by this approach (e.g. cervix, lung) and very high sensitivity and specificity can be achieved approaching or even surpassing those achieved by human experts using atypical cell detection approach.

## Claims

1. An apparatus for detecting malignancy associated changes in cells including a microscope having a high sampling density light transducer optically connected to the primary image plane, and an imaging board associated with the transducer to capture and process digitized images from the light transducer, characterized in that the computer controls the microscope and imaging board to detect malignancy associated changes by:
(a) controlling the microscope to acquire a precise and reproducible focus of a nucleus by maximizing contrast of the nuclear material as a function of focal position;
(b) obtaining an image of a cell nucleus;
(c) correcting the image of the cell nucleus for imperfections caused by the microscope;
(d) comparing the pixels in the corrected image with a threshold to obtain a first approximation of the real edge of the nucleus;
(e) determining a real edge of the nucleus by step by step removal of pixels with the least gradient so as not to break a continuous contour around the nucleus;
(f) measuring nuclear features of the cell nucleus in the corrected image; and
(g) analyzing the measured features using a multivariate analysis to detect the presence of malignancy associated changes.

2. The apparatus as in claim 1 wherein the computer adjusts the location of edges of the nuclei as a function of each feature to be analyzed.

3. The apparatus as in claim 1 wherein the multivariate analysis comprises a cluster analysis leading to a decision tree composed of thresholds and discriminant functions.

4. The apparatus as in claim 1 wherein the multivariate analysis comprises a neural network.

5. A method of detecting malignancy associated changes in cells comprising the steps of:
(a) providing a microscope having a high sampling density light transducer optically connected to the primary image plane of the microscope and an imaging board for processing images from the light transducer;
(b) acquiring precise and reproducible focus of a nucleus in the image from the microscope by maximizing contrast of the nuclear material as a function of focal position;
(c) thresholding the image such that the threshold mask includes at least all of the edges of the nucleus to obtain a first approximation of the real edge of the nucleus;
(d) determining the real edge of the nucleus by step by step removal of pixels with the least gradient so as not to break a continuous contour around the nucleus;
(e) analysing features of the image including DNA distribution of the cell nuclei; and
(f) performing multivariate analysis of the image of the nucleus comprising cluster analysis leading to a decision tree composed of thresholds and discriminant functions.

6. A method as in claim 5 comprising the additional step of adjusting the location of the edges of the nucleus as a function of each feature to be analysed.

7. A method of detecting malignancy associates changes in cells comprising the steps of :
(a) providing a microscope having a high sampling density light transducer optically connected to the primary image plane of the microscope and an imaging board for processing images from the light transducer;
(b) acquiring precise and reproducible focus of a nucleus in the image from the microscope by maximizing contrast of the nuclear material as a function of focal position;
(c) thresholding the image such that the threshold mask includes at least all of the edges of the nucleus to obtain a first approximation of the real edge of the nucleus;
(d) determining the real edge of the nucleus by step by step removal of pixels with the least gradient so as not to break a continuous contour around the nucleus;
(e) analysing features of the image including DNA distribution of the cell nuclei; and
(f) performing multivariate analysis of the image of the nucleus comprising a neural network.

8. A method as in claim 7 comprising the additional step of adjusting the location of the edges of the nucleus as a function of each feature to be analyzed.

## Patentansprüche

1. Vorrichtung zum Erkennen von mit Bösartigkeit verbundenen Veränderungen in Zellen, welche ein Mikroskop mit einem Lichtwandler mit hoher Abtastdichte, der mit der Primärbildebene optisch verbunden ist, und eine zum Wandler gehörige Abbildungsplatine, um digitalisierte Bilder vom Lichtwandler zu erfassen und zu verarbeiten, umfaßt; dadurch gekennzeichnet, daß der Computer das Mikroskop und die Abbildungsplatine steuert, um mit Bösartigkeit verbundene Veränderungen zu erkennen, durch:
(a) Steuern des Mikroskops, um einen präzisen und reproduzierbaren Brennpunkt eines Zellkerns zu erfassen, durch Maximieren des Kontrasts des Zellkernmaterials als Funktion der Brennpunktlage;
(b) Erhalten eines Bildes eines Zellkerns;
(c) Korrigieren des Bildes des Zellkerns hinsichtlich Mängeln, die durch das Mikroskop verursacht werden;
(d) Vergleichen der Pixel im korrigierten Bild mit einem Schwellenwert, um eine erste Näherung der tatsächlichen Kante des Zellkerns zu erhalten;
(e) Bestimmen einer tatsächlichen Kante des Zellkerns durch schrittweise Entfernung von Pixeln mit dem geringsten Gradienten, um eine durchgehende Kontur um den Zellkern nicht zu unterbrechen;
(f) Messen von Zellkernmerkmalen des Zellkerns im korrigierten Bild; und
(g) Analysieren der gemessenen Merkmale unter Verwendung einer Mehrvariablenanalyse, um die Anwesenheit von mit Bösartigkeit verbundenen Veränderungen zu erkennen.

2. Vorrichtung nach Anspruch 1, wobei der Computer den Ort der Kanten der Zellkerne als Funktion jedes zu analysierenden Merkmals einstellt.

3. Vorrichtung nach Anspruch 1, wobei die Mehrvariablenanalyse eine Clusteranalyse umfaßt, die zu einem Entscheidungsbaum führt, der aus Schwellenwerten und Unterscheidungsfunktionen besteht.

4. Vorrichtung nach Anspruch 1, wobei die Mehrvariablenanalyse ein Nervennetzwerk umfaßt.

5. Verfahren zum Erkennen von mit Bösartigkeit verbundenen Veränderungen in Zellen, welches die Schritte umfaßt:
(a) Bereitstellen einem Mikroskops mit einem Lichtwandler mit hoher Abtastdichte, der mit der Primärbildebene des Mikroskops optisch verbunden ist, und einer Abbildungsplatine zum Verarbeiten von Bildern vom Lichtwandler;
(b) Erfassen eines präzisen und reproduzierbaren Brennpunkts eines Zellkerns in dem Bild vom Mikroskop durch Maximieren des Kontrasts des Zellkernmaterials als Funktion der Brennpunktlage;
(c) Schwellenwertbestimmung im Bild, so daß die Schwellenwertmaske zumindest alle Kanten des Zellkerns enthält, um eine erste Näherung der tatsächlichen Kante des Zellkerns zu erhalten;
(d) Bestimmen der tatsächlichen Kante des Zellkerns durch schrittweise Entfernung von Pixeln mit dem geringsten Gradienten, um eine durchgehende Kontur um den Zellkern nicht zu unterbrechen;
(e) Analysieren von Merkmalen des Bildes, einschließlich der DNA-Verteilung der Zellkerne; und
(f) Durchführen einer Mehrvariablenanalyse des Bildes des Zellkerns, welche eine Clusteranalyse umfaßt, die zu einem Entscheidungsbaum führt, der aus Schwellenwerten und Unterscheldungsfunktionen besteht.

6. Verfahren nach Anspruch 5, welches den zusätzlichen Schritt der Einstellung des Orts der Kanten des Zellkerns als Funktion jedes zu analysierenden Merkmals umfaßt.

7. Verfahren zum Erkennen von mit Bösartigkeit verbundenen Veränderungen in Zellen, welches die Schritte umfaßt:
(a) Bereitstellen eines Mikroskops mit einem Lichtwandler mit hoher Abtastdichte, der mit der Primärbildebene des Mikroskops optisch verbunden ist, und einer Abbildungsplatine zum Verarbeiten von Bildern vom Lichtwandler;
(b) Erfassen eines präzisen und reproduzierbaren Brennpunkt eines Zellkerns in dem Bild vom Mikroskop durch Maximieren des Kontrasts des Zellkernmaterials als Funktion der Brennpunktlage;
(c) Schwellenwertbestimmung im Bild, so daß die Schwellenwertmaske zumindest alle Kanten des Zellkerns enthält, um eine erste Näherung der tatsächlichen Kante des Zellkerns zu erhalten;
(d) Bestimmen der tatsächlichen Kante des Zellkerns durch schrittweise Entfernung von Pixeln mit dem geringsten Gradienten, um eine durchgehende Kontur um den Zellkern nicht zu unterbrechen;
(e) Analysieren von Merkmalen des Bildes, einschließlich der DNA-Verteilung der Zellkerne; und
(f) Durchführen einer Mehrvariablenanalyse des Bildes des Zellkerns, welche ein Nervennetzwerk umfaßt.

8. Verfahren nach Anspruch 7, welches den zusätzlichen Schritt der Einstellung des Orts der Kanten des Zellkerns als Funktion jedes zu analysierenden Merkmals umfaßt.

## Revendications

1. Appareil pour la détection de changements associés à la malignité dans des cellules, comprenant un microscope doté d'un phototransducteur à haute densité d'échantillonnage optiquement relié au plan de l'image primaire, et un dispositif d'imagerie associé au transducteur pour saisir et traiter les images numérisées à partir du phototransducteur ; caractérisé en ce que l'ordinateur règle le microscope et le dispositif d'imagerie afin de détecter les changements associés à la malignité :
(a) en réglant le microscope afin d'obtenir une mise au point précise et reproductible d'un noyau en maximisant le contraste du matériel nucléaire en fonction de la distance focale ;
(b) en obtenant une image d'un noyau cellulaire ;
(c) en corrigeant les imperfections de l'image du noyau cellulaire dues au microscope ;
(d) en comparant les pixels dans l'image corrigée avec un seuil afin d'obtenir une première approximation du bord réel du noyau ;
(e) en déterminant un bord réel du noyau en éliminant pas à pas les pixels présentant le plus faible gradient de façon à ne pas interrompre un contour continu autour du noyau ;
(f) en mesurant les caractéristiques nucléaires du noyau cellulaire dans l'image corrigée ; et
(g) en analysant les caractéristiques nucléaires mesurées au moyen d'une analyse multivariée afin de détecter la présence de changements associés à la malignité.

2. Appareil selon la revendication 1, dans lequel l'ordinateur ajuste la position des bords des noyaux en fonction de chaque caractéristique à analyser.

3. Appareil selon la revendication 1, dans lequel l'analyse multivariée comprend une analyse de groupes (cluster) permettant de tracer un arbre décisionnel composé de seuils et de fonctions discriminantes.

4. Appareil selon la revendication 1, dans lequel l'analyse multivariée comprend un réseau neuronal.

5. Procédé de détection de changements associés à la malignité dans des cellules comprenant les étapes consistant à :
(a) fournir un microscope doté d'un phototransducteur à haute densité d'échantillonnage optiquement relié au plan de l'image primaire du microscope et un dispositif d'imagerie pour traiter les images du phototransducteur ;
(b) obtenir une mise au point précise et reproductible d'un noyau dans l'image provenant du microscope en maximisant le contraste du matériel nucléaire en fonction de la distance focale ;
(c) réaliser un seuillage de l'image de telle façon que la fenêtre de seuillage comprenne au moins tous les bords du noyau pour obtenir une première approximation du bord réel du noyau ;
(d) déterminer le bord réel du noyau en éliminant pas à pas les pixels présentant le plus faible gradient de façon à ne pas interrompre un contour continu autour du noyau ;
(e) analyser les caractéristiques de l'image, y compris la distribution de l'ADN dans les noyaux cellulaires ; et
(f) réaliser une analyse multivariée de l'image du noyau comprenant une analyse de groupes (cluster) permettant de tracer un arbre décisionnel composé de seuils et de fonctions discriminantes.

6. Procédé selon la revendication 5, comprenant l'étape supplémentaire d'ajustement de la position des bords du noyau en fonction de chaque caractéristique à analyser.

7. Procédé de détection de changements associés à la malignité dans des cellules comprenant les étapes consistant à :
(a) fournir un microscope doté d'un phototransducteur à haute densité d'échantillonnage optiquement relié au plan de l'image primaire du microscope et un dispositif d'imagerie pour traiter les images du phototransducteur ;
(b) obtenir une mise au point précise et reproductible d'un noyau dans l'image provenant du microscope en maximisant un contraste du matériel nucléaire en fonction de la distance focale ;
(c) réaliser un seuillage de l'image de telle façon que la fenêtre de seuillage comprenne au moins tous les bords du noyau pour obtenir une première approximation du bord réel du noyau ;
(d) déterminer le bord réel du noyau en éliminant pas à pas les pixels présentant le plus faible gradient de façon à ne pas interrompre un contour continu autour du noyau ;
(e) analyser les caractéristiques de l'image, y compris la distribution de l'ADN dans les noyaux cellulaires ; et
(f) réaliser une analyse multivariée de l'image du noyau comprenant un réseau neuronal.

8. Procédé selon la revendication 7, comprenant l'étape supplémentaire d'ajustement de la position des bords du noyau en fonction de chaque caractéristique à analyser.
